# EUROPEAN PATENT APPLICATION

(11) **EP 1 731 169 A1**
(43) Date of publication of application: **13.12.2006**
(21) Application number: 05720356.4
(22) Date of filing: 09.03.2005
(51) Int. Cl.: A61K 45/00, A61K 31/4164, A61P 13/08

(54) **REMEDIES FOR PROSTATIC HYPERTROPHY**

(30) Priority: 12.03.2004 JP 2004069946
(71) Applicant: Yokohama TLO Company, Ltd., Yokohama-shi, Kanagawa 240-8501 (JP)
(72) Inventor: KUBOTA, Yoshinobu, Yokohama-shi, Kanagawa 236-0031 (JP); UEMURA, Hiroji, Yokohama-shi, Kanagawa 240-0011 (JP)
(74) Representative: Weihs, Bruno Konrad
(86) International application number: PCT/JP2005/004084
(87) International publication number: WO 2005/087267

(57) **Abstract**

A treatment and/or preventive agent for prostatic hypertrophy includes a compound having an antagonistic effect to an angiotensin II receptor, or a pharmaceutically acceptable salt or a prodrug thereof, as an active ingredient, wherein said compound having an antagonistic effect to an angiotensin II receptor is one or more compounds selected from the group consisting of losartan, potassium losartan, candesartan, candesartan cilexetil, balsartan, eprosartan, zolasartan, telmisartan, irbesartan, tasosartan, olmesartan medoxomil, and olmesartan.

## Description

### TECHNICAL FIELD

The present invention relates to compounds and compositions for treating and/or preventing prostatic hypertrophy, compounds and compositions for inhibiting the proliferation of prostate cells, and the usage thereof. More generally, the present invention relates to compounds and compositions for inhibiting the phosphorylation of MAP kinase (MAPK) in normal cells of the prostate gland, and a method of using the same.

### BACKGROUND ART

Prostatic hypertrophy occurs in men as early as in their 40s, and most men experience it by their 60s. Prostatic hypertrophy is caused by the increase of some stromal and/or epithelial cells constituting the prostate. However, unlike cancer, the cell proliferation is regulated to stop at a definite point. Therefore, prostatic hypertrophy is a benign disease which will not result in death by itself, but its accompanying symptoms such as dysuresia significantly affect the quality of life (QOL) of the patients.

Prostatic hypertrophy had been treated mainly by surgery such as abdominal surgery (laparotomy) and transurethral prostatectomy (TUR-P), but since the 1990s, drug treatment with α1 receptor blockers, antiandrogenic agents, crude drugs, Chinese medicines, or other drugs is becoming more common.

α1 receptor blockers have been widely used because they can improve dysuresia associated with prostatic hypertrophy by relaxing muscular strain in the urethra or other parts with fewer side effects. However, these agents can not reduce developed prostatic adenomas. On the other hand, antiandrogenic agents can improve the symptom by reducing the developed glandular epithelium. However, the patients must continue taking the agents, otherwise the adenomas return to the size before the treatment. Moreover, the agents cause the deterioration of the ability for erection due to their antiandrogenic effect. Crude drugs and Chinese medicines may be used for relatively mild cases or for cases in which other drugs has failed or will fail to improve the symptoms. However, their working mechanism has not been fully elucidated, and their effects may be placebo effects.

Angiotensin II mediates physiological actions such as vascular constriction through AT1 receptors. Therefore, angiotensin II receptor-antagonistic drugs, in particular AT1 receptor-antagonistic drugs, have been used as antihypertensive or treatment agents for circulatory system diseases such as heart diseases. Recently, an angiotensin II receptor-antagonistic drug has been found to have an inhibitory effect on the proliferation of prostatic cancer cells (Nonpatent Document 1: Uemura, H. et al., "Angiotensin II receptor blocker shows antiproliferative activity in prostate cancer cells: A possibility of tyrosine kinase inhibitor of growth factor," Mol. Cancer Ther., 2003; 2:1139-1147; Patent Document 1: Japanese Patent Application Laid-Open (JP-A) No. 2004-2282). However, there is no description in these references as to whether the angiotensin II receptor-antagonistic drug has any effect on the proliferation of non-cancerous normal cells of the prostate gland or on the treatment and/or prevention of prostatic hypertrophy.
[Patent Document 1] Japanese Patent Application Laid-Open (JP-A) No. 2004-2282
[Nonpatent Document 1] Uemura, H. et al., "Angiotensin II receptor blocker shows antiproliferative activity in prostate cancer cells: A possibility of tyrosine kinase inhibitor of growth factor," Mol. Cancer Ther., 2003; 2:1139-1147

### DISCLOSURE OF THE INVENTION

### PROBLEM TO BE SOLVED BY THE INVENTION

The present invention is intended to provide novel preventive and/or treatment agents for prostatic hypertrophy and proliferation inhibitors for normal cells of the prostate gland, more generally, compounds and compositions for inhibiting the MAPK phosphorylation in normal cells of the prostate gland, which are capable of suppressing the excessive proliferation of normal cells in the prostate gland, which are effective against both of hypertrophy due to the proliferation of epithelial cells and hypertrophy due to the proliferation of stromal cells, which cause little side effects, and can be used alone or in combination with other drugs used in conventional drug therapy, as well as a method for using the same.

The inventors of the present invention have found that compounds having an antagonistic effect to angiotensin II receptors suppress the proliferation of non-cancerous cells of the prostate gland, and they have accomplished the present invention.

More specifically, the present invention provides:
(1) a treatment and/or preventive agent for prostatic hypertrophy comprising a compound having an antagonistic effect to an angiotensin II receptor, or a pharmaceutically acceptable salt or a prodrug thereof, as an active ingredient;
(2) the treatment and/or preventive agent according to the above (1), wherein the compound having an antagonistic effect to an angiotensin II receptor is a non-peptide type angiotensin II receptor-antagonistic compound;
(3) the treatment and/or preventive agent according to the above (1) or (2), wherein the compound having an antagonistic effect to an angiotensin II receptor is a compound having an imidazole skeleton or imidazole-related skeleton of any of imidazole-based, benzimidazole-based, imidazopyridine-based, triazole-based, pyridine-based, methoxypyridine-based, pyrazole-based, pyrrole-based, azaindene-based, pyrimidone-based, quinazoline-based, xanthine-based, condensed imidazole-based, pyrimidine dione-based or thienopyridone-based;
(4) the treatment and/or preventive agent according to any of the above (1) to (3), wherein the compound having an antagonistic effect to an angiotensin II receptor is one or more compounds selected from the group consisting of losartan, potassium losartan, candesartan, candesartan cilexetil, balsartan, eprosartan, zolasartan, telmisartan, irbesartan, tasosartan, olmesartan medoxomil, and olmesartan;
(5) a medicament comprising a combination of the treatment and/or preventive agent according to any of the above (1) to (4) and a treatment and/or preventive agent for prostatic hypertrophy containing one or more active ingredients selected from the group consisting of α1 blocking agents, antiandrogenic agents, plant extracts and Chinese medicines, anticholinergic agents, and amino acid preparations;
(6) an inhibitor for the proliferation of normal cells of the prostate gland comprising a compound having an antagonistic effect to an angiotensin II receptor, or a pharmaceutically acceptable salt or a prodrug thereof, as an active ingredient;
(7) the inhibitor for the proliferation according to the above (6), wherein the normal cells of the prostate gland are stromal cells and/or epithelial cells;
(8) an inhibitor for the MAPK phosphorylation in normal cells of the prostate gland, comprising a compound having an antagonistic effect to an angiotensin II receptor, or a pharmaceutically acceptable salt or a prodrug thereof, as an active ingredient;
(9) the inhibitor for the MAPK phosphorylation according to the above (8), wherein the normal cells of the prostate gland are stromal cells and/or epithelial cells; and
(10) the inhibitor for the MAPK phosphorylation according to the above (8) or (9), wherein the MAPK phosphorylation is caused by angiotensin II, or a growth factor or a cytokine.

With regard to the present invention,"normal" cells refer to non-cancerous cells, and do not mean that the cells contain no abnormality for the rest.

The present invention further provides a method for using the above mentioned drugs.

### EFFECTS OF THE INVENTION

The compounds and compositions of the present invention for the treatment and/or prevention of prostatic hypertrophy, inhibitors for the proliferation of prostate cells, and/or compounds and compositions for inhibiting the MAPK phosphorylation in normal cells of the prostate gland are capable of suppressing the excessive proliferation of normal cells in the prostate gland, and effective against both hypertrophy due to the proliferation of epithelial cells and hypertrophy due to the proliferation of stromal cells. They cause little side-effects, and can be used alone or in combination with other drugs used in conventional drug therapy.

Accordingly, by using the treatment and/or preventive agent for prostatic hypertrophy, inhibitors for the proliferation of cells of the prostate gland, and/or the compounds and compositions for inhibiting the MAPK phosphorylation in normal cells of the prostate gland, of the present invention, one can safely reduce the hypertrophy without surgery while avoiding problems such as side-effects inherent in conventional drug therapy. As a result, uncomfortable symptoms due to prostatic hypertrophy can be improved by removing the cause, and thereby QOL of the patients can be improved.

### BRIEF DESCRIPTION OF THE DRAWINGS

Fig. 1 shows the effect of angiotensin II and candesartan cilexetil (CV11974; hereinafter may be abbreviated as"CV" or"candesartan") on the proliferation of PrSC cells, a cell line derived from normal human prostatic stromal cells.
Fig. 2 shows the effect of TGFβ and CV on the proliferation of PrSC cells.
Fig. 3 shows the effect of EGF and CV on the proliferation of PrSC cells.
Fig. 4 shows the effect of TGFβ, TNFα and CV on the secretion of IL-6 by PrSC cells.
Fig. 5 shows the concentration-dependent effect of EGF and the effect of CV on the secretion of IL-6 by PrSC cells.
Fig. 6 shows the concentration-dependent effect of angiotensin II and the effect of CV on the secretion of IL-6 by primary-cultured F-1 cells.
Fig. 7 shows the effect of TNFα and CV on the secretion of IL-6 by primary-cultured FT-FB cells.
Fig. 8 shows the effect of CV in vivo on PC-3 tumor grafts transplanted on mice.
Fig. 9 shows the effect of CV in vivo on mixed tumor grafts of PC-3 cells and PrSC cells transplanted on mice.
Fig. 10 shows the inhibitory effect of olmesartan and TNFα on the proliferation of PrSC cells.
Fig. 11 shows the inhibitory effect of telmisartan and EGF or TNFα on the proliferation of PrSC cells.
Fig. 12 shows the concentration-dependent inhibitory effect of losartan at various concentrations on the proliferation of PrSC cells.
Fig. 13 shows the inhibitory effect of losartan on the proliferation of TNFα-stimulated PrSC cells.
Fig. 14 shows the inhibitory effects of AIIR blockers (candesartan, losartan) on the secretion of paracrine factors from PrSC cells, and on the MAPK phosphorylation in PC-3 cells. Panels (A) and (B) show the results of Western blotting of pMAPK and MAPK, respectively.
Fig. 15 shows the inhibitory effect of AIIR blockers (candesartan, losartan) on the MAPK phosphorylation in PrSC cells. Panels (A) and (B) show the results of Western blotting of pMAPK and MAPK, respectively.

### BEST MODE FOR CARRYING OUT THE INVENTION

With regard to the present invention, "an antagonistic effect for an angiotensin II receptor" refers to the property of inhibiting the development of physiological actions of angiotensin II by interfering with the binding of angiotensin II to an angiotensin II receptor, in particular to an AT1 receptor.

Typical examples of the compounds having an antagonistic effect for an angiotensin II receptor (hereinafter may be referred to as "an angiotensin II receptor-antagonistic agent") include those described below.

Angiotensin II receptor-antagonistic agents are broadly divided into peptide and non-peptide types, or classified into competitive and uncompetitive types according to the inhibition of angiotensin II-induced vascular constriction. The compounds having an antagonistic effect for an angiotensin II receptor for use in the present invention may be any of them.

For example, examples of the non-peptide type angiotensin II receptor-antagonistic agents include biphenyltetrazole-based and non-biphenyltetrazole-based compounds; each group of compounds includes those having an imidazole skeleton or an imidazole-related (analogue) skeleton.

More specifically, various compounds are known such as those imidazole-based, benzimidazole-based, imidazopyridine-based, triazole-based, pyridine-based, methoxypyridine-based, pyrazole-based, pyrrole-based, azaindene-based, pyrimidone-based, quinazoline-based, xanthine-based, condensed imidazole-based, pyrimidine dione-based, and thienopyridone-based compounds.

Further more specific examples include losartan (Dup753), potassium losartan (chemical name: 2-butyl-4-chloro-1-[2'-(tetrazole-5-yl)biphenyl-4-ylmethyl]-1H-imidazole-5-methanol potassium salt), candesartan, candesartan cilexetil (TCV-116; chemical name: 1-(cyclohexyloxycarbonyloxy)ethyl 2-ethoxy-1-[[2'-(1H-tetrazole-5-yl)biphenyl-4-yl]methyl]-1H-benzimidazole-7-carboxylate), balsartan (CGP-48933; chemical name: N-{4-[2-(1H-tetrazole-5-yl)phenyl]benzyl}-N-valeryl-valine), eprosartan (SK&F108566), zolasartan (GR-117289), telmisartan (BIBR277; chemical name: 4'-{[4-methyl-6-(1-methyl-2-benzimidazolyl)-2-propyl-1-benzimidazolyl]methyl}-2-biphenylcarboxylic acid), irbesartan (SR47436), tasosartan (ANA-736), and olmesartan medoxomil (CS-866; chemical name: 2,3-dihydroxy-2-butenyl 4-(1-hydroxy-1-methylethyl)-2-propyl-1-[p-(o-1H-tetrazole-5-ylphenyl)benzyl]imidazole-5-carboxylate, cyclic 2,3-carbonate).

Examples of a "pharmaceutically acceptable salt" of the above compounds include metal salts such as sodium, potassium, calcium, and magnesium; salts with amines such as triethylamine, trimethylamine, ethanolamine, diethanolamine, triethanolamine, dicyclohexylamine, N, N'-dibenzylethylenediamine, pyridine, and picoline; and salts with basic amino acids such as lysine, arginine, and ornithine.

With regard to the present invention, a prodrug of an angiotensin II receptor-antagonistic agent is a compound that is converted in vivo into an active metabolite (angiotensin II receptor-antagonistic agent). Examples thereof include derivatives modified by acylation, alkylation, phosphorylation, eicosanoylation, alanylation, pentyl aminocarbonylation, (5-methyl-2-oxo-1,3-dioxolen-4-yl)methoxycarbonylation, tetrahydrofuranylation, pyrrolidylmethylation, pivaloyloxymethylation, tert-butylation and so on, of an amino group; acylation, alkylation, phosphorylation, boration, acetylation, palmitoylation, propanoylation, pivaloylation, succinylation, fumarylation, alanylation, dimethylaminomethylcarbonylation and so on, of a hydroxy group; and esterification, ethylesterification, phenylesterification, carboxymethylesterification, dimethylaminomethylesterification, pivaloyloxymethylesterification, ethoxycarbonyloxyethylesterification, phthalidylesterification, (5-methyl-2-oxo-1,3-dioxolen-4-yl)methylesterification, cyclohexyloxycarbonyloxyethylesterification, methylamidation and so on, of a carboxyl group.

As described above, the angiotensin II receptor-antagonistic agent according to the present invention may be in a salt or ester form. Hereinafter, compounds having an antagonistic effect to an angiotensin II receptor, and pharmaceutically acceptable salts and prodrugs thereof may be collectively referred to as an "AIIR blocker(s)".

The treatment and/or preventive agents of the present invention may be solely composed of one, or two or more types of AIIR blockers, or maybe in the form of a pharmaceutical composition containing other ingredients in addition to an AIIR blocker. Examples of these additional ingredients include pharmaceutically acceptable various carriers, excipients, diluents, stabilizing agents, binding agents, disintegrating agents, solvents, buffering agents, suspending agents, emulsifying agents, preservatives, fragrant materials, sweeteners, and colorants, which are known in the technical field of pharmaceutics. These ingredients may be appropriately selected by those skilled in the art according to the planned administration route, dosage form, and the like.

The route of administration may be oral or parenteral. Examples of administration routes include oral, pernasal, percutaneous, intrarectal, subcutaneous, intracutaneous, intramuscular, intraperitoneal, and intravenous routes.

Examples of the dosage forms include tablets, powders, granules, sugar-coated tablets, capsules, syrups, suspensions, emulsified liquids and the like for oral administration; liquids for injection, depots, intravenous drips, nasal drips and the like; and ointments, gels, liquids, suppositories, patches and the like for external use. The method for manufacturing these various preparations is known to those skilled in the art. For example, the treatment and/or preventive agents of the present invention can be manufactured in the same manner as various angiotensin II receptor blocker-containing preparations, which have been manufactured or used as an antihypertensive or a treatment agent for circulatory system diseases such as heart diseases, and various preparations as stated in Patent Document 1.

Furthermore, the AIIR blockers according to the present invention may be combined with other drugs for suppressing prostate hypertrophy or for relieving the symptoms of prostatic hypertrophy, or other drugs. The treatment and/or preventive agents of the present invention may contain these components, or they may be administered to the same patient simultaneously or sequentially in separate formulations. Examples of the drugs which can be combined include α1 blocking agents such as blazocine hydrochloride, tamsulosin hydrochloride, urapidil and so on; antiandrogenic agents such as chlormadinone acetate, allyl estrenol and so on; vegetable preparations such as eviprostat and plant extracts composing such preparations, cernitin pollen extract, plant extracts or Chinese medicines such as Chorei-to; and other drugs such as anticholinergic drugs and amino acid preparations used for relieving the symptoms associated with prostate hypertrophy.

The treatment and/or preventive agents of the present invention contain an AIIR blocker in an amount effective for achieving their medical purposes (pharmaceutically effective amount). Specific amount is determined by a known method using an appropriate animal model or the like. The amount of the AIIR blocker as an active ingredient usually ranges, when orally administered 1 to 3 doses per day, 0.01 µg/kg body weight to 10 mg/kg body weight, with 1 µg/kg body weight to 5 mg/kg body weight being preferred.

The precise dose and administration schedule are determined individually in accordance with the characteristics of the treated individual (for example, age, body weight, sex, the degree of infection or disease, and so on), the route of administration, other drugs or medical treatments to be combined, duration in the body of a specific preparation, and other factors.

### EXAMPLES

| Example of preparation 1 : Preparation of tablet | |
|---|---|
| Candesartan cilexetil | 50 mg |
| Lactose | 35 mg |
| Corn starch | 130 mg |
| Microcrystalline cellulose | 30 mg |
| Magnesium stearate | 5 mg |
| Total | 250 mg |

Powders of the above formulation are mixed, and the resulting mixture is compressed into a 250 mg tablet using a tablet machine.

### Example 1: Inhibitory effect on the proliferation of prostatic stromal cells stimulated with angiotensin II

A cell line derived from normal human prostatic stromal cells, i.e., PrSC cells, was purchased from Sanko Junyaku Co. Ltd. (Normal Human Prostate Stromal Cells, catalogue number CC-2508), and maintained by subculturing them in a phenol red-free RPMI medium under conditions of 5% CO₂ and 37°C.

The PrSC cells (10⁴ cells) were placed in a 12-well dish, and cultured in a phenol red-free medium containing 0.1% (W/V) BSA under conditions of 5% CO₂ and 37°C for several days. Then, 1 µM or 10 µM of angiotensin II was added. To some wells, CV (10 µM; hereinafter CV was used at this concentration unless otherwise noted) was added as an AIIR blocker 4 hours before adding angiotensin II. Each group contained four wells (n=4). After culturing the cells at 5% CO₂ and 37°C for 5 days, the number of all cells in each of the wells was counted.

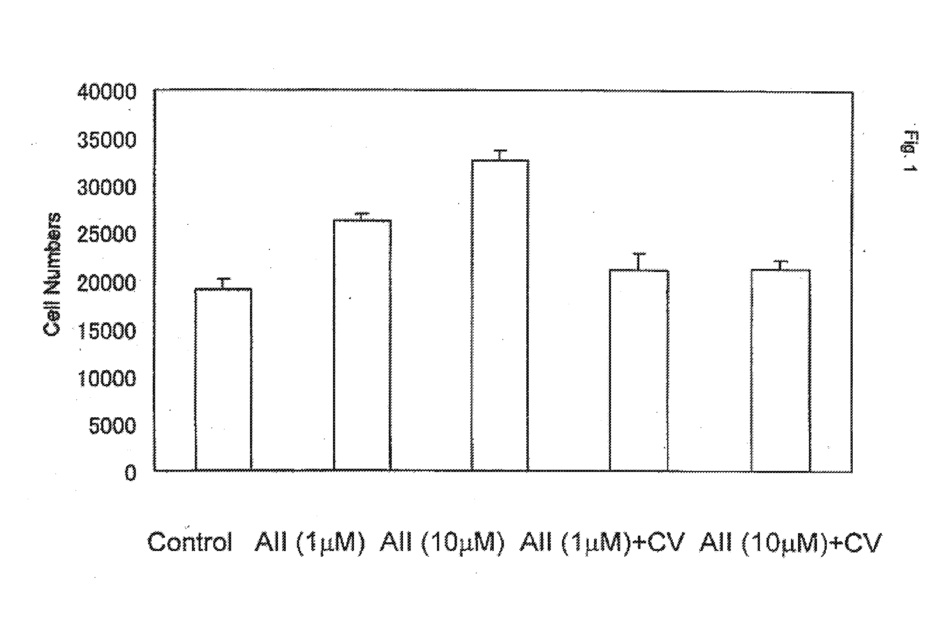

The results are shown in Fig. 1. In comparison with the control group ("Control") receiving no angiotensin II, the groups stimulated with angiotensin II ("AII (1 µM)" and"AII (10 µM)") showed a concentration-dependent increase in the numbers of cells. On the other hand, the increase in the numbers of cells of the groups added with CV before being stimulated with angiotensin II ("AII (1 µM) + CV" and "AII (10 µM) + CV") were suppressed to a level similar to that of the control group. Accordingly, highly significant differences were found between "AII (1 µM)" and "AII (1 µM) + CV", and between "AII (10 µM)" and "AII (10 µM) + CV" (P<0.01).

### Example 2: Inhibitory effect on the proliferation of prostatic stromal cells stimulated with TGFβ

The effect of an AIIR blocker was examined in the same manner as Example 1, except for that 10 ng/mL of TGFβ was used in place of angiotensin II.

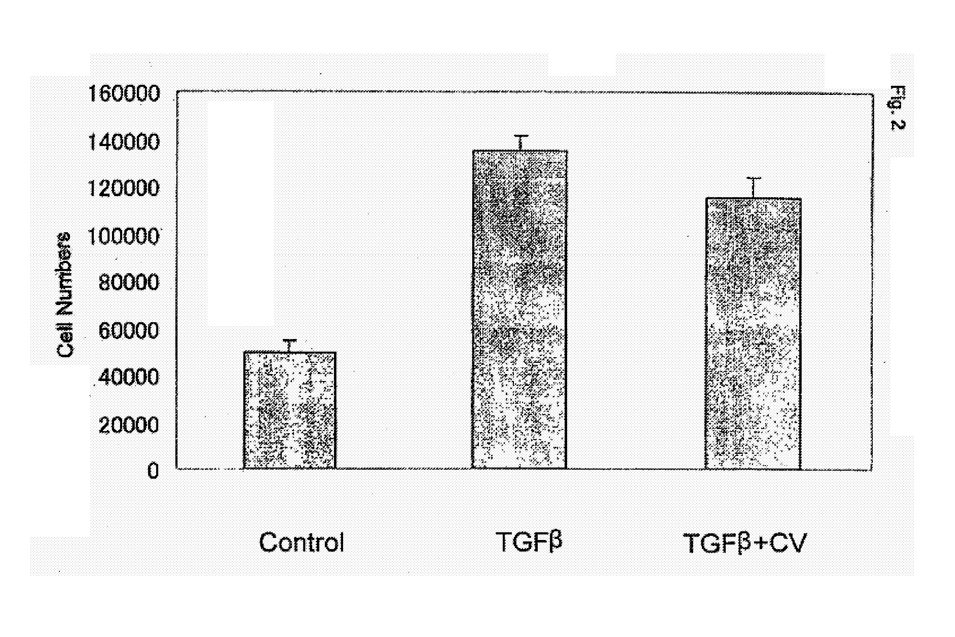

The results are shown in Fig. 2. The number of cells of the group stimulated with TGFβ ("TGFβ") showed a more than 2-fold increase as compared with that of the control group receiving no TGFβ ("Control"). On the other hand, the increase in the number of cells of the group added with 10 µM of CV before being stimulated with TGFβ ("TGFβ + CV") was significantly suppressed in comparison with that of "TGFβ" (P<0.05).

### Example 3: Inhibitory effect on the proliferation of prostatic stromal cells stimulated with EGF

The effect of an AIIR blocker was examined in the same manner as Example 1, except for that 10 ng/mL of EGF was used in place of angiotensin II.

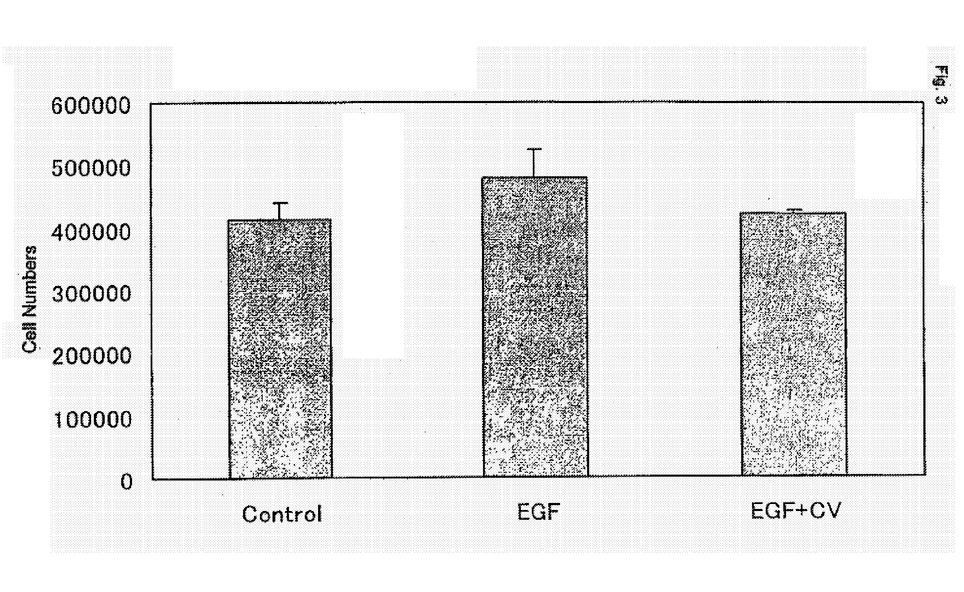

The results are shown in Fig. 3. The number of cells of the group stimulated with EGF ("EGF") showed a significant increase as compared with that of the control group receiving no EGF ("Control"). On the other hand, the increase in the number of cells of the group added with CV before being stimulated with EGF ("EGF + CV") was significantly suppressed (P<0.05) to the same level as that of the control group.

### Example 4: Inhibitory effect on IL-6 secretion by prostatic stromal cells

The primary-cultured stromal cells (F-1 cells) derived from a patient with prostatic hypertrophy were prepared as described below.

Tissues obtained from the patient were placed in a 50 mL centrifuge tube containing 20 mL of a medium (RPMI 1640, GIBCO 11835-030) supplemented with 10% (V/V) FBS (filtron) and mg/mL penicillin/streptomycin (GIBCO 15140-122), and allowed to stand at 4°C for 1 hour or longer. Subsequently, the tissues were placed in a 10-cm dish, and cut into pieces of about 1 mm square. Blood vessels and the like were removed. At that time, to remove excessive blood (if necessary), the tissues were washed by adding 20 mL of the medium and centrifuging the mixture for 5 minutes at 1,000 rpm, followed by aspiration of the supernatant.

The obtained tissue pieces were placed in a centrifuge tube containing 5 mL of a collagenase solution (1 mg/mL of collagenase type II (SIGMA C-6885) in a medium of the same composition as the above-described medium, except that RPMI 1640 GIBCO 11835-030 was replaced with RPMI 1640 GIBCO 31800-022), to which 20 mL of the medium was added, and the mixture was stirred for 1.5 to 3 hours at 37°C.

Subsequently, the mixture was centrifuged at 1,000 rpm for 5 minutes, and the supernatant was aspirated. The same operation was repeated two to three times to wash the pellets with the medium. At that time, excessive suspended matters were removed by filtration, if necessary.

The obtained cells were plated out on a 10-cm dish, and the proliferated cells obtained 1 to 2 weeks after plating were used as the primary culture.

The primary cells (FT-FB cells) derived from the stromal cells of another patient with prostatic hypertrophy were prepared by the basically same procedure as described above.

PrSC cells prepared in the same manner as Example 1, or F-1 or FT-FB cells prepared as described above were placed in a 6-well dish at 10⁵ cells per well, cultured at 5% CO₂ and 37°C for 1 day (24 hours), and stimulated by the addition of TGFβ (1 ng/mL), TNFα (1 ng/mL), EGF (2.5 or 10 ng/mL), or angiotensin II (1 µM or 10 µM) (n=3). CV was added as the AIIR blocker to some wells 4 hours before adding the reagents. The culture media were collected 24 hours after adding the reagents, and the concentrations of IL-6 were determined by the CLEIA method.

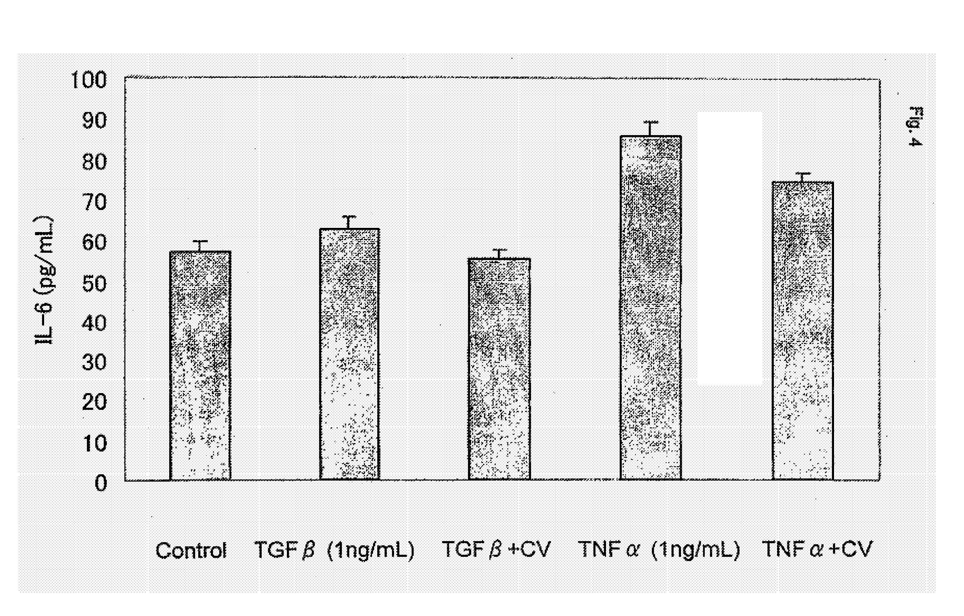

The results are shown in Figs. 4 to 7. Fig. 4 shows the results of the stimulation of PrSC cells with TGFβ or TNFα. IL-6 secretion of the groups stimulated with TGFβ or TNFα ("TGFβ (1 ng/mL)" and "TNFα (1ng/mL)", respectively) was significantly promoted in comparison with that of the control group ("Control") receiving no TGFβ or TNFα. On the other hand, IL-6 secretion of the groups to which CV was added before the stimulation ("TGFβ + CV" and "TNFα + CV") was significantly suppressed (P<0.04 and P<0.01, respectively) in comparison with that of "TGFβ (1 ng/mL)" and "TNFα (1 ng/mL)", respectively.

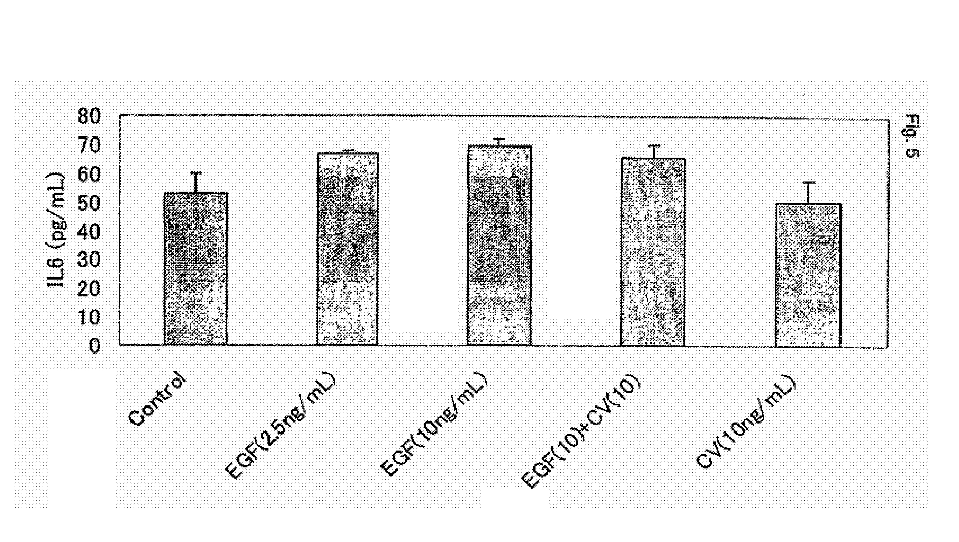

Fig. 5 shows the results of the stimulation of PrSC cells with EGF. IL-6 secretion of the groups stimulated with EGF ("EGF (2.5 ng/mL)" and "EGF(10 ng/mL)") showed a dose-dependent increase in comparison with that of the control group ("Control") receiving no EGF. On the other hand, IL-6 secretion of the group to which CV was added before the stimulation ("EGF (10) + CV (10)") was significantly suppressed (P<0.05) in comparison with that of "EGF (10 ng/mL)".

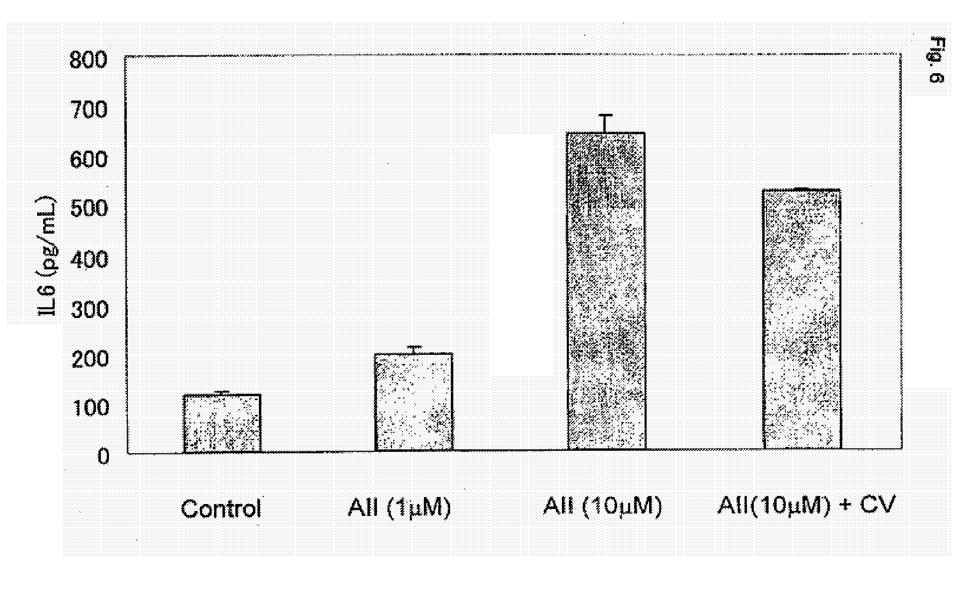

Fig. 6 shows the results of the stimulation of F-1 cells with angiotensin II. IL-6 secretion of the groups stimulated with angiotensin II ("AII(1 µM)" and "AII (10 µM)") showed a significant increase in a concentration-dependent manner in comparison with that of the control group ("Control") receiving no angiotensin II. On the other hand, IL-6 secretion of the group to which CV was added before the stimulation ("AII (10 µM)"+CV") was significantly suppressed (P<0.03) in comparison with that of "AII (10 µM)".

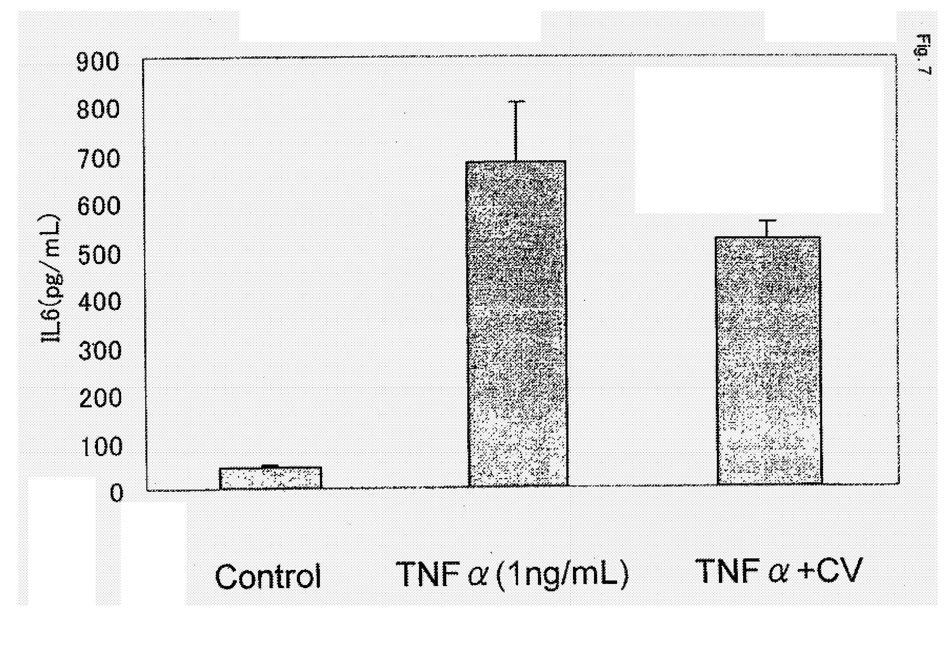

Fig. 7 shows the results of the stimulation of FT-FB cells with TNFα. IL-6 secretion of the group stimulated with TNFα ("TNFα (1 ng/mL)") increased about 12 to 15 times in comparison with that of the control group ("Control") receiving no TNFα. On the other hand, IL-6 secretion of the group to which CV was added before the stimulation ("TNFα + CV") was significantly suppressed (P=0.051) in comparison with that of "TNFα (1 ng/mL)".

The results of above Examples have revealed that while prostatic stromal cells stimulated with a cell growth factor secretes IL-6, the AIIR blocker can suppress the secretion of IL-6 and thereby suppressing the IL-6-induced proliferation of epithelial cells of the prostate gland.

Accordingly, the AIIR blocker can suppress the proliferation of both the normal stromal and epithelial cells of the prostate gland.

### Example 5: In vivo inhibitory effect on proliferation of prostate cells

The in vivo effect of the AIIR blocker was examined using the PC-3 human prostatic cancer cells with less expression of AT1 receptors (obtained from American Type Culture Collection, Rockville, MD, USA), and PrSC cells.

PC-3 cells (5×10⁶ cells) or a mixture of PC-3 and PrSC cells (2.5×10⁶ cells each; a total of 5×10⁶ cells) were injected subcutaneously in the back of male nude mice of 4 weeks old for forming tumors. Tumors having a diameter of about 5 mm were formed after one week.

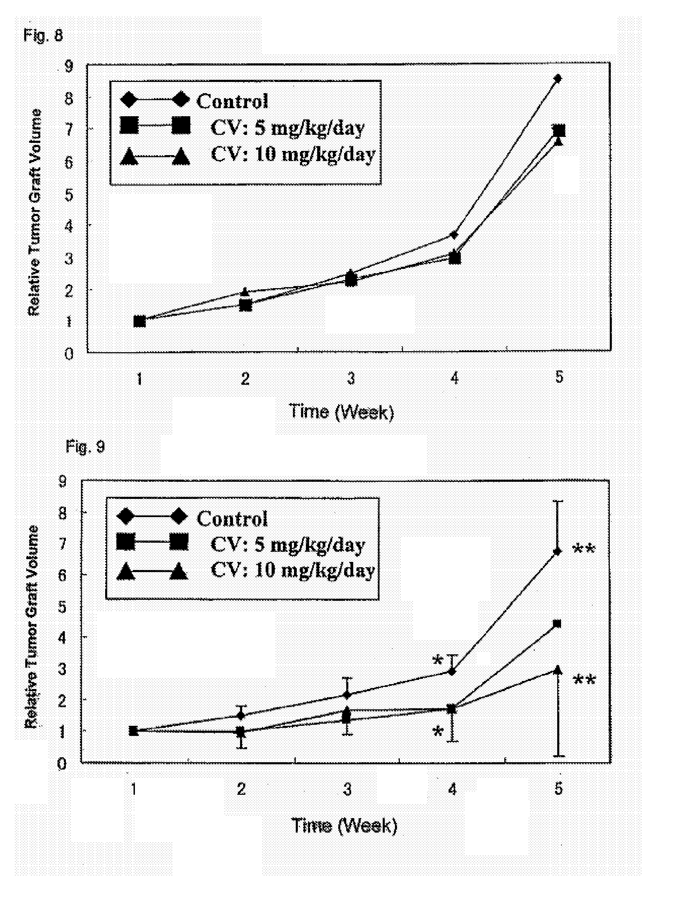

The above-described mice were divided into three groups: CV (5 mg/kg/day) administered group, CV (10 mg/kg/day) administered group, and non-administered group (Control) (n=4 for each group). The administered groups were orally administered with a specified dose of CV dissolved in drinking water every day from the first to the fifth week after the tumorigenic injection. The size of the tumor (heterograft) on each mouse was measured every week, and the volume was calculated using the formula: major axis × minor axis × minor axis / 2. The volume was expressed as relative ratio to the volume in the first week as being 1. The results are shown in Figs. 8 (PC-3 cells were transplanted) and 9 (a mixture of PC-3 and PrSC cells was transplanted).

Since PC-3 cells have fewer expressed AT1 receptors in comparison with other prostatic cancer cells (DU145 and LNCaP), the AIIR blocker was expected to have a lower inhibitory effect on their proliferation. The result shown in Fig. 8 indicates that, as predicted above, the suppression of proliferation was observed for the CV administered groups as compared to "Control", but the effect was not so remarkable. On the other hand, when the mixture of PC-3 and PrSC cells was transplanted (Fig. 9), the proliferation in the administered groups was dose-dependently suppressed in comparison with that in "Control", and significant differences were observed between "Control" and "CV: 10 mg/kg/day" in the fourth and fifth weeks (P<0.04("*") and P<0.03 ("**"), respectively).

Accordingly, it was revealed that the AIIR blocker suppressed in vivo the proliferation of stromal cells. Further, it was shown that the AIIR blocker not only directly suppressed the proliferation of prostatic cancer cells but also suppressed the proliferation of prostatic cancer cells through the effects on stromal cells, such as inhibition of the secretion of growth factors, cytokine or the like.

### Example 6: Inhibitory effect of olmesartan on proliferation of prostatic stromal cells

PrSC cells were plated out on a 12-well dish at 20,000 cells per well, and cultured for 5 days in a phenol red-free F12 culture medium supplemented with 0.1% (W/V) BSA containing TNFα (1 ng/mL) alone or in combination with olmesartan (1 µM). Then the number of cells was counted. The group with no additive was used as the control group. Each group contained four wells (n=4).

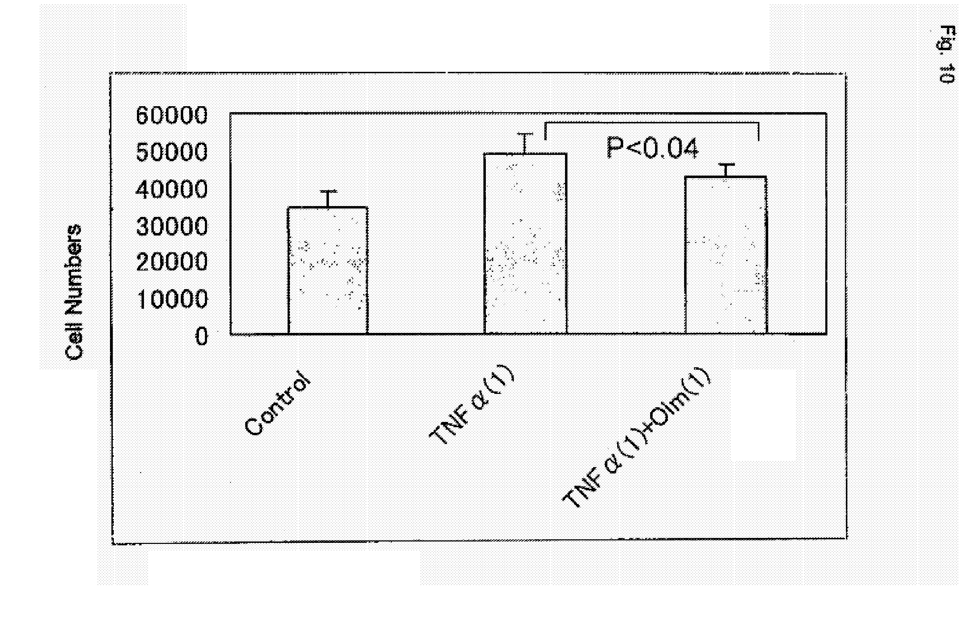

The results are shown in Fig. 10. The number of cells of the group stimulated with TNFα ("TNFα (1)") significantly increased in comparison with that of the control group ("Control") (P<0.01). On the other hand, the increase in the number of cells of the group to which the combination of TNFα and olmesartan was added ("TNFα (1) + Olm (1)") was significantly suppressed (P<0.04).

### Example 7: Inhibitory effect of telmisartan on proliferation of prostatic stromal cells

PrSC cells were plated out on a 12-well dish at 25,000 cells per well, and cultured for 5 days in a phenol red-free F12 culture medium supplemented with 0.1% (W/V) BSA containing EGF (10 ng/mL) or TNFα (1 ng/mL), each alone or in combination with telmisartan (50 µM). Then the number of cells was counted. The group with no additive was used as the control group. Each group contained four wells (n=4).

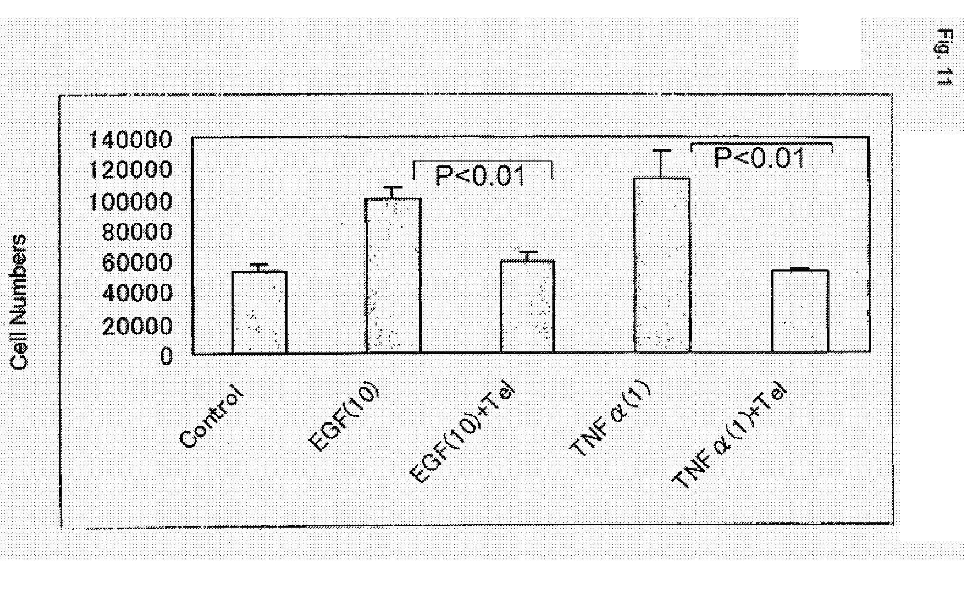

The results are shown in Fig. 11. The number of cells of the groups stimulated with EGF or TNFα ("EGF (10)" or "TNFα (1)", respectively) significantly increased in comparison with that of the control group ("Control") (P<0.01). On the other hand, the increase in the number of cells of the groups to which telmisartan was added at the same time ("EGF (10) + Tel" and "TNFα (1) + Tel") was significantly suppressed (P<0.01).

### Example 8: Inhibitory effect of losartan on proliferation of prostatic stromal cells (1)

PrSC cells were plated out on a 12-well dish at 20,000 cells per well, and cultured for 5 days in a phenol red-free F12 culture medium containing 10% (V/V) FCS and losartan at various concentrations (0, 0.1, 1.0, and 10 µM). Then the number of cells was counted. The group with no additive was used as the control group. Each group contained four wells (n=4).

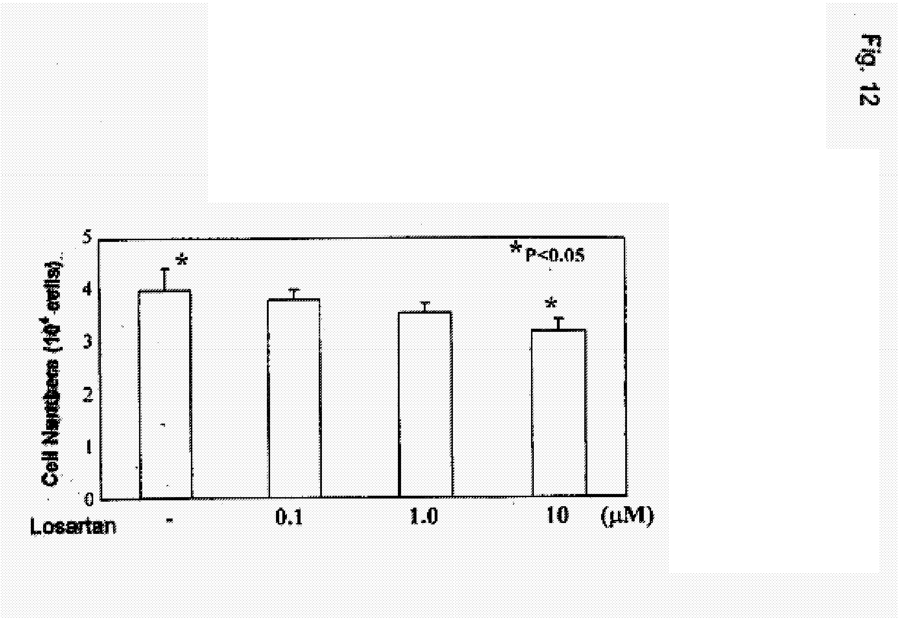

The results are shown in Fig. 12. The cell proliferation of the group to which losartan (0.1, 1.0, and 10 µM) was added was dose-dependently suppressed in comparison with that of the control group (losartan"-"), and the group to which 10 µM of losartan was added showed a significant difference in comparison with the control group (P<0.05).

### Example 9: Inhibitory effect of losartan on proliferation of prostatic stromal cells (2)

PrSC cells were plated out on a 12-well dish at 25,000 cells per well, and cultured for 5 days in a phenol red-free F12 culture medium supplemented with 0.1% (W/V) BSA containing TNFα (1 ng/mL) and/or losartan (10 µM). Then the number of cells was counted. The group with no additive was used as the control group. Each group contained four wells (n=4).

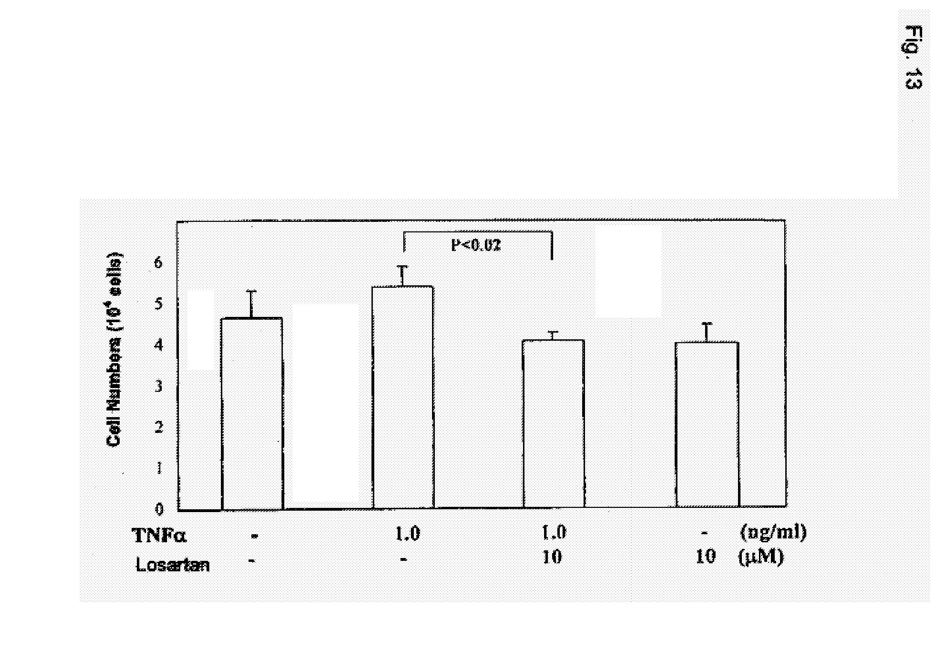

The results are shown in Fig. 13. The number of cells of the group stimulated with TNFα (TNFα/losartan "1.0/-") significantly increased in comparison with that of the control group (TNFα/losartan "-/-") (P<0.05). On the other hand, the increase in the number of cells of the group to which the combination of TNFα and losartan was added (TNFα/losartan"1.0/10") was significantly suppressed (P<0.02). Furthermore, the cell proliferation of the group to which losartan alone was added (TNFα/losartan "-/10") showed no change, and was equivalent to that of the control group.

### Example 10: Inhibition of paracrine factor secretion from stromal cells by AIIR blocker

### (10-1)

Stromal cells (PrSC) were cultured in a phenol red-free F12 medium containing 0.1% (W/V) BSA, to which angiotensin II (10 µM) was added alone or in combination with losartan ("Los"; 10 µM). The culture medium was collected 24 hours after the addition, and used as a conditioned medium ("0.1% BSA-CM").

### (10-2)

Stromal cells (PrSC) were cultured in a phenol red-free F12 medium containing 10% (V/V) FCS, to which CV (10 µM) or losartan (10 µM) was added. The culture medium was collected 24 hours after the addition, and used as a conditioned medium ("10% FCS-CM") with no addition of angiotensin II. 10% FCS-CM with no additive was also prepared in the same manner.

3,000,000 PrSC cells were used for each of the above 10-1 and 10-2.

PC3 cells (prostate cancer cells) were cultured for 24 hours in a phenol red-free F12 medium supplemented with 0.1% (W/V) BSA. Then the medium was replaced with one of the conditioned media prepared as described above, and the cells were collected 10 minutes later. Total cellular proteins were extracted as described below. Similar protocol was done in parallel for PC-3 cells without using any conditioned medium ("Control").

The cells were washed twice with an ice-cold phosphate buffer (PBS), and lysed in 200 µL of an ice-cold cell lysis solution (lysis buffer; 20 mM of Tris (pH8.0), 137 mM of NaCl, 10% of glycerol, 0.1% of SDS, 0.5% of Nonidet P-40 (trade name), 100 mM of sodium fluoride, 200 mM of sodium orthovanadate, 1mM of EGTA, 2 mM of phenylmethylsulfonyl fluoride, 1 mg/mL of leupeptin, and 3 mg/mL of aprotinin). The mixture was centrifuged. Subsequently, the supernatant was collected.

The resulting protein sample was subjected to SDS-PAGE in the routine manner, and the MAPK phosphorylation was determined by Western blotting using an anti-phospho-MAP kinase antibody or an anti-MAP kinase antibody) (both were purchased from Cell Signaling Technology (Beverly, MA)) as the detecting antibody.
From the obtained results, the expression levels of pMAPK and MAPK bands were determined using NIH image software. On the basis of the results, the pMAPK/MAPK ratio in each lane was calculated, and then the ratio ("pMAPK ratio") of the obtained value of each lane to that of the lane 1 ("Control") was calculated by taking the value of Control as 1.

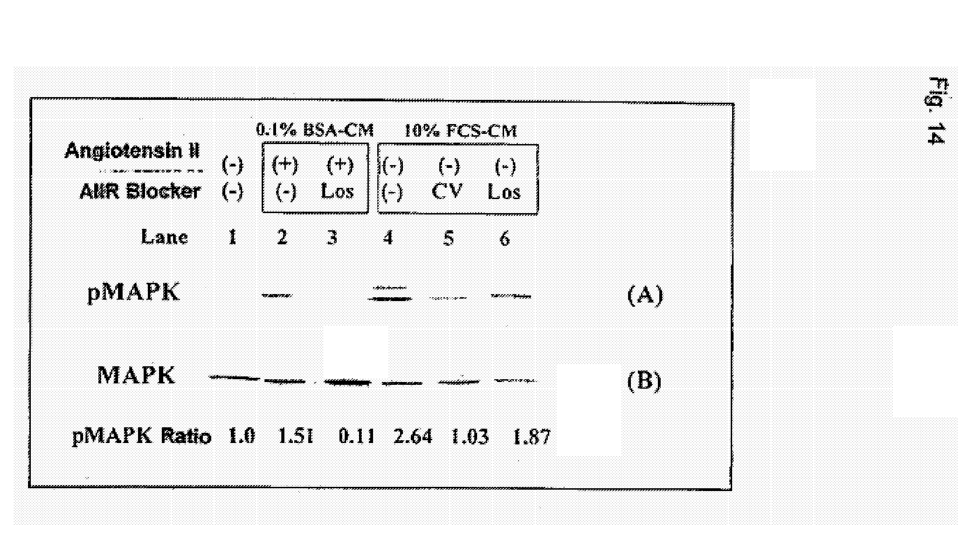

The results are shown in Fig. 14. The mechanism of the proliferation of prostate cells (epithelial and stromal cells) has been considered as follows: various growth factors and/or cytokines bind to the receptors in the cell membrane to transmit the proliferation signals into the cell, during which the phosphorylation of MAPK, a member of the proliferation signal transduction pathway, occurs and the signals are transmitted to downstream of the proliferation cascade to finally result in the production of proteins in the proliferation system. In this experiment, the MAPK phosphorylation in PC-3 cells was more remarkable in the lane 4 wherein the cells were cultured in the conditioned medium prepared by culturing PrSC cells with no additive than in the lane 1 wherein the cells were cultured without using any conditioned medium. This fact validates that the stromal cells secrete growth factors and/or cytokines (paracrine factors) such as IL-1, IL-6, IL-8, MCP-1 and MCSF, thereby promote the MAPK phosphorylation and cell proliferation in PC-3 cells.

Further, the MAPK phosphorylation in PC-3 cells was caused when the PrSC conditioned medium containing angiotensin II alone was used in culture (lane 2), but the phosphorylation was suppressed when the conditioned medium containing angiotensin II and losartan were used in culture (lane 3). Furthermore, the MAPK phosphorylation in PC-3 cells was caused when the conditioned medium with no additive was used in culture (lane 4), but the phosphorylation was suppressed when the conditioned medium containing CV or losartan was used in culture (lanes 5 and 6).
These results indicate that the AIIR blockers suppress the secretion of paracrine factors from stromal cells, thereby suppressing the proliferation of prostatic epithelial cells.

### Example 11: Inhibition of MAPK phosphorylation in prostatic stromal cells by AIIR blocker

Stromal cells (PrSC) were cultured for 24 hours in a phenol red-free F12 medium containing 0.1% (W/V) BSA, to which angiotensin II (10 µM) or TNFα (1 ng/mL) was added each alone or in combination with either CV (10 µM) or losartan ("Los"; 10 µM) by replacing the medium. The cells were collected 10 minutes after the addition, and the total cellular proteins were extracted in the same manner as described in Example 10. Similarly, the cells cultured with no additive and the cells added with either CV or Los alone were processed in parallel. 3,000,000 PrSC cells were used for each sample.
These samples were subjected to Western blotting in the same manner as described in Example 10.

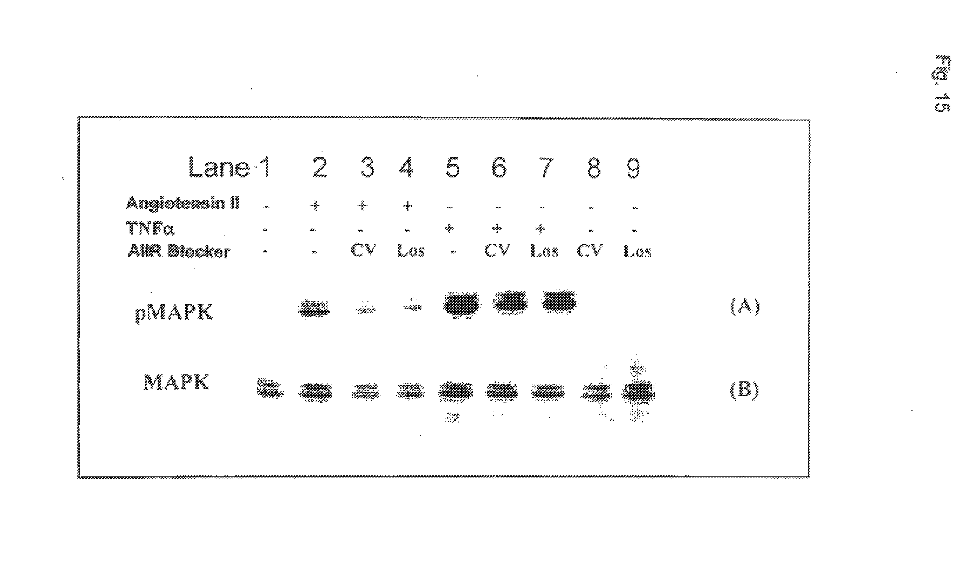

The results are shown in Fig. 15. The MAPK phosphorylation was strongly activated when angiotensin II alone was added (lane 2), but was suppressed when angiotensin II was added in combination with CV or Los (lanes 3 and 4). Similar results were obtained when TNFα was added; the MAPK phosphorylation was strongly activated when TNFα alone was added (lane 5), but was suppressed when TNFα was added in combination with CV or Los (lanes 6 and 7).
When either CV or Los was added alone, the MAPK phosphorylation was not caused (lanes 8 and 9).
These results indicate that the AIIR blockers act on stromal cells of the prostate gland to suppress the transduction of proliferation signals involving angiotensin II and TNFα.

The present application is based on Japanese Patent Application 2004-069946, a Japanese patent application filed on March 12, 2004, and the contents described in the Specification and Claims of Japanese Patent Application 2004-069946 are all included in the Specification of the present application.

## Claims

1. A treatment and/or preventive agent for prostatic hypertrophy comprising a compound having an antagonistic effect to an angiotensin II receptor, or a pharmaceutically acceptable salt or a prodrug thereof, as an active ingredient.

2. The treatment and/or preventive agent according to claim 1, wherein the compound having an antagonistic effect to an angiotensin II receptor is a non-peptide type angiotensin II receptor-antagonistic compound.

3. The treatment and/or preventive agent according to claim 1 or 2, wherein the compound having an antagonistic effect to angiotensin II receptors is a compound having an imidazole skeleton or imidazole-related skeleton of any of imidazole-based, benzimidazole-based, imidazopyridine-based, triazole-based, pyridine-based, methoxypyridine-based, pyrazole-based, pyrrole-based, azaindene-based, pyrimidone-based, quinazoline-based, xanthine-based, condensed imidazole-based, pyrimidine dione-based or thienopyridone-based.

4. The treatment and/or preventive agent according to any one of claims 1 to 3, wherein the compound having an antagonistic effect to an angiotensin II receptor is one or more compounds selected from the group consisting of losartan, potassium losartan, candesartan, candesartan cilexetil, balsartan, eprosartan, zolasartan, telmisartan, irbesartan, tasosartan, olmesartan medoxomil, and olmesartan.

5. A medicament comprising a combination of the treatment and/or preventive agent according to any one of claims 1 to 4 and a treatment and/or preventive agent for prostatic hypertrophy containing one or more active ingredients selected from the group consisting of α1 blocking agents, antiandrogenic agents, plant extracts and Chinese medicines, anticholinergic agents, and amino acid preparations.

6. An inhibitor for the proliferation of normal cells of the prostate gland comprising a compound having an antagonistic effect to an angiotensin II receptor, or a pharmaceutically acceptable salt or a prodrug thereof, as an active ingredient.

7. The inhibitor for the proliferation according to claim 6, wherein the normal cells of the prostate gland are stromal cells and/or epithelial cells.

8. An inhibitor for the MAPK phosphorylation in normal cells of the prostate gland, comprising a compound having an antagonistic effect to an angiotensin II receptor, or a pharmaceutically acceptable salt or a prodrug thereof, as an active ingredient.

9. The inhibitor for the MAPK phosphorylation according to claim 8, wherein the normal cells of the prostate gland are stromal cells and/or epithelial cells.

10. The inhibitor for the MAPK phosphorylation according to claim 8 or 9, wherein the MAPK phosphorylation is caused by angiotensin II, or a growth factor or a cytokine.
